# EUROPEAN PATENT APPLICATION

(11) **EP 4 843 635 A1**
(43) Date of publication of application: **25.11.2026**
(21) Application number: 26180009.8
(22) Date of filing: 20.05.2026
(51) Int. Cl.: A63B 21/002, A63B 21/062, A63B 21/072, A63B 21/078, A61B 5/00, A61B 5/11, A61B 5/22, A63B 24/00

(54) **A MUSCLE STRENGTH MEASURING DEVICE**

(30) Priority: 21.05.2025 SE 2530297
(71) Applicant: Augustsson, Jesper, 39363 Kalmar (SE)
(72) Inventor: Augustsson, Jesper, 39363 Kalmar (SE)
(74) Representative: Swea IP Law AB

(57) **Abstract**

The present invention relates to a muscle strength measuring device (1). The muscle strength measuring device comprises a barbell (5), a base unit (9), a plurality of rods (3) extending vertically from the base unit and arranged to receive the barbell, two sensor units (7) arranged between the base unit and the rods and being configured to measure a force applied to the barbell, and a holding arrangement (11) for holding the barbell in a fixed vertical position with respect to the base unit during measuring of the force applied to the barbell. The holding arrangement (11) comprises a locking arrangement (13) movable along the rods and arranged to lock the barbell against the rods in any desired vertical position to enable adjustment of the barbell along the rods. The device comprises a control unit (23) is configured to receive measured force data from sensor units (7).

## Description

### Technical field

The present invention generally relates to a muscle strength measuring device, and in particular to an improved muscle strength measuring device capable of measuring a force applied to a barbell.

### Background

Manual isometric muscle testing is a common clinical technique that is used to assess muscle strength. Isometric Mid-Thigh Pull (IMTP) is primarily a measure of lower body muscle strength and function. The most common IMTP tests today are when a test person performs the test using a squatting cage or using other IMTP testing equipment where the test person stands on one or two force plates in a specially adapted stand. The stand has adjustable barbell heights to allow the test person to adjust their lifting position.

There is also a form of IMTP equipment that consists of a bar and a force cell, attached to a chain in the floor or substrate. By reducing or increasing the number of links in the chain, the lifting position of the test person can be adjusted.

There are several problems with current IMTP tests which are listed below:
Methodological problems:
   - The test person stands on force plates, which makes it difficult to define the start of the test. This in turn makes it difficult to measure early force production, i.e. explosive strength. Using a bar, force cell, e.g. force plate, and chain is neither any good in this regard, as either a slack or a pretension can occur that makes it impossible to define the start of the test. The ability to assess maximum strength can also be impaired for the same reason. According to conventional methods for assessing early force production, uni-directional force plates are employed, wherein the test person is positioned standing on the force plates. This arrangement causes the test person's body mass to be integrated into the measured force output. Following a start signal, the subject commences a pulling motion on a bar. Accurate detection of force onset is challenging in such setups, as the inclusion of body mass and baseline fluctuations may obscure the initial change in force output.
   - It is not possible to reliably assess unilateral force development and thus asymmetries, i.e. differences in muscle strength between the right and left halves of the body, because the body weight distributed over the two halves of the body is unknown when the test subject stands on two force plates. Using a bar, force cell and chain is neither any good in this regard, as force is measured with a single force cell, which makes it impossible to distinguish between the left and right halves of the body.
   - Regardless of the test, it is not possible to adjust the distance between the barbell and the floor with sufficient precision, which affects reliability and the ability to measure in the test person's most optimal lifting position.
Ethical problem:
   - An ethical dilemma may arise when using force plates, as the test person's body weight becomes visible, which can be sensitive in a situation where a group of people are being tested.
Economic problem:
   - Force plates vary in price but are generally expensive.
Practical problem:
   - Portability (the ability to move the equipment) is relatively limited, as force plates and poles are both heavy and bulky (force plates weigh at least 13-18 kg).

KR20230090760 A discloses a muscle strength measuring device comprising a pair of frames which are described as being approximately 'c' shaped. The barbell bar height is adjusted by inserting it into one of a "plurality of insertion holes" located along the height of the frame plate. KR20230090760 A provides a device having a step-wise height adjustment system and a sensing module provided between the frame and the base. The entire C-shaped frame rests on this sensing module. This mechanical arrangement places the sensor under the entire frame structure.

There is thus a need for a new improved testing equipment capable of enabling a test that overcomes the problems associated with current IMTP tests.

### Summary

An object of the present invention is to provide an improved device capable of measuring a force applied to a barbell.

This aim is achieved by a muscle strength measuring device as defined in claim 1.

The muscle strength measuring device comprises a horizontal barbell, a horizontal base unit and a plurality of rods extending vertically from the base unit and arranged to receive the barbell. The muscle strength measuring device further comprises two sensor units arranged between the base unit and the rods and being configured to measure a force applied to the barbell. The muscle strength measuring device further comprises a holding arrangement for holding the barbell in a fixed vertical position with respect to the base unit during measuring of the force applied to the barbell. The holding arrangement comprises a locking arrangement movable along the rods and arranged to lock the barbell against the rods in any desired vertical position to enable adjustment of the barbell along the rods.

Such a muscle strength measuring device enables a cheaper, lighter, more compact and more portable solution. Such a muscle strength measuring device also provides a particularly useful solution to fine-adjusting the barbell along the rods.

Each sensor unit comprises a load cell. Using load cells is beneficial as load cells cost significantly less than e.g. force plates.

The load cells are bi-directional load cells such that they measure both tension forces and compression forces.

In contrast to force plates, which are typically uni-directional and configured to measure compression forces only, the load cells according to the invention are bi-directional and configured to measure both tension and compression forces. This overcomes the critical difficulty of accurately measuring explosive strength by eliminating ambiguity from slack or baseline fluctuations.

The muscle strength measuring device further comprises a control unit connected to the sensor units. The control unit is configured to receive measured force data from the sensor units. The control unit is configured to determine a zero-force crossing point that defines the start of a test, based on the force transitioning from negative to positive. The control unit may further comprise one or more processors and a memory storing software instructions which, when executed by the processor(s). The software instructions may further be configured to determine the force at predefined time points based on the measured data and the determined zero-force crossing point.

The control unit is configured to:
receive measured force data from the sensor units,
determine a zero-force crossing point, which defines the start of a test, based on the force transitioning from negative to positive, and
wherein the zero-force crossing point is determined based on that a positive force is preceded by a negative force.

According to an embodiment, the control unit is configured to determine the force at predefined time points based on the measuring data and the determined zero-force crossing point.

The device of the invention is uniquely constructed with two sensor units arranged directly between the vertical support rods and the base unit. Force is transmitted down the rods and measured at their base. This configuration enables reliable, independent measurement of force from the left and right sides of the body, allowing for the assessment of muscular asymmetries. It also completely isolates the force measurement from the user's body weight.

The dual-sensor design enables the measurement of strength asymmetries, a key diagnostic metric not reliably offered by the prior art.

The device of the invention is lightweight and compact and thus portable.

The invention replaces expensive force plates with relatively inexpensive load cells thus reducing costs.

By measuring force applied to the barbell independently of the user's body weight, the device resolves both the ethical and methodological problems associated with systems that integrate body mass into the measurement.

According to an embodiment, the adjustment of the barbell along the rods is continuous. This allows for continuous and highly precise adjustment of the barbell's height, enabling a user to find their most biomechanically optimal lifting position.

According to an embodiment, at least one of the rods is threaded and the locking arrangement is provided with at least one opening with corresponding threads.

According to an embodiment, said plurality of rods comprises a first set of rods and a second set of rods arranged at a horizontal distance from each other, and the rods in each of said first and second sets of rods are arranged spaced apart so that a space is formed between them for receiving the barbell, and wherein the holding arrangement comprises:
- a first holding device arranged movable along the first set of rods, and
- a second holding device arranged movable along the second set of rods, wherein each of the first and second holding devices defines an opening between the rods of said first and second sets of rods for receiving one end of the barbell.

According to an embodiment, the locking arrangement is arranged to lock the first and second holding devices against the first and second sets of rods respectively in any desired vertical position.

According to an embodiment, at least one of the first set of rods and the second set of rods are threaded and wherein the locking arrangement is provided with at least one opening with corresponding threads.

According to an embodiment, the barbell comprises one or more openings for receiving the rods.

According to an embodiment, at least one of the plurality of rods is smooth.

The invention is based on the realisation that, by providing a holding arrangement comprising a locking mechanism that is movable along the rods and configured to lock the barbell in any desired vertical position, highly precise adjustment of the barbell along the rods is enabled, thereby improving the functionality of the device.

### Brief description of the drawings

The invention will now be explained more closely by the description of different embodiments of the invention and with reference to the appended figures.
- Fig. 1: shows schematically an example of a muscle strength measuring device in a frontal view according to some embodiments.
- Fig. 2: shows another example of the muscle strength measuring device in a frontal view according to some embodiments.
- Fig. 3: shows another example of the muscle strength measuring device in a frontal view according to some embodiments.
- Fig. 4: shows an example of a part of a muscle strength measuring device in a frontal view according to an embodiment.
- Fig. 5: shows another example of a muscle strength measuring device in a frontal view according to some embodiment.
- Fig. 6a and 6b: shows other examples of a muscle strength measuring device in a frontal view according to an embodiment.
- Fig.7a and 7b: shows other examples of a muscle strength measuring device according to some embodiments.
- Fig. 8: shows an example of a procedure according to an embodiment.

It should be noted that the drawings have not necessarily been drawn to scale and that the dimensions of certain features may have been exaggerated for the sake of clarity.

Any feature of any embodiment of the device depicted or described in any part of this document may be combined with any one or more features of any other embodiment, unless such a combination is explicitly excluded herein.

### Detailed description

Aspects of the present disclosure will be described more fully hereinafter with reference to the accompanying drawings. The coupling device can, however, be realized in many different forms and should not be construed as being limited to the aspects set forth herein. Like numbers in the drawings refer to like elements throughout.

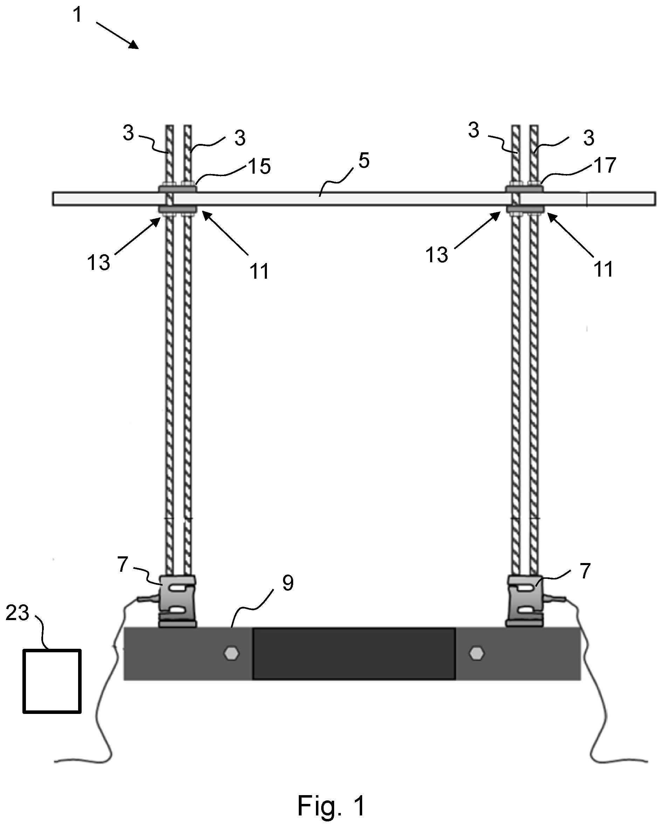

**Figure 1** shows an example of a muscle strength measuring device 1 in a frontal view. The muscle strength measuring device 1 comprises a barbell 5. The muscle strength measuring device 1 further comprises a base unit 9 such as a platform. The muscle strength measuring device 1 further comprises a plurality of rods 3 extending vertically from the base unit 9 and arranged to receive the barbell 5. In the example in figure 1 there are four rods 3 with two rods on each side, however there may also be one rod 3, e.g. a single rod, on each side extending vertically from the base unit 9 and arranged to receive the barbell 5. In the example in figure 1 the rods 3 are threaded. The rods 3 may have a circular, square, rectangular, hexagonal, octagonal, triangular, elliptical, I-beam, T-shaped, L-shaped, star-shaped, or spline cross-section.

The muscle strength measuring device 1 further comprises two sensor units 7 arranged between the base unit 9 and the rods 3 and being configured to measure a force applied to the barbell 5. Each of the sensor units 7 comprises a load cell. The load cells are bi-directional load cells such that they measure both tension forces and compression forces.

The muscle strength measuring device 1 further comprises a holding arrangement 11 for holding the barbell in a fixed vertical position with respect to the base unit 9 during measuring of the force applied to the barbell 5.

The holding arrangement 11 comprises a locking arrangement 13 movable along the rods 3 and arranged to lock the barbell 5 against the rods 3 in any desired vertical position to enable adjustment of the barbell 5 along the rods 3. Thereby a very precise adjustment of the barbell 5 along the rods 3 is enabled. The adjustment of the barbell 5 along the rods 3 may be stepwise or continuous. The locking arrangement 13 may comprise one or more screw-nuts, one or more knobs, and/or one or more stop rings or spring clips. The holding arrangement 11 may comprise one or more joint brackets. In the example in figure 1 with two threaded rods 3 on each side, the locking arrangement 13 may comprise one or more screw nuts.

The muscle strength measuring device 1 may comprise one or more control units 23 connected to the sensor units 7. The connection between the control unit 23 and the sensor units 7 may be wireless or wired.

According to an embodiment, at least one of the rods 3 may be threaded and wherein the locking arrangement 13 may be provided with at least one opening with corresponding threads. According to an embodiment, at least one of the rods 3 may be smooth.

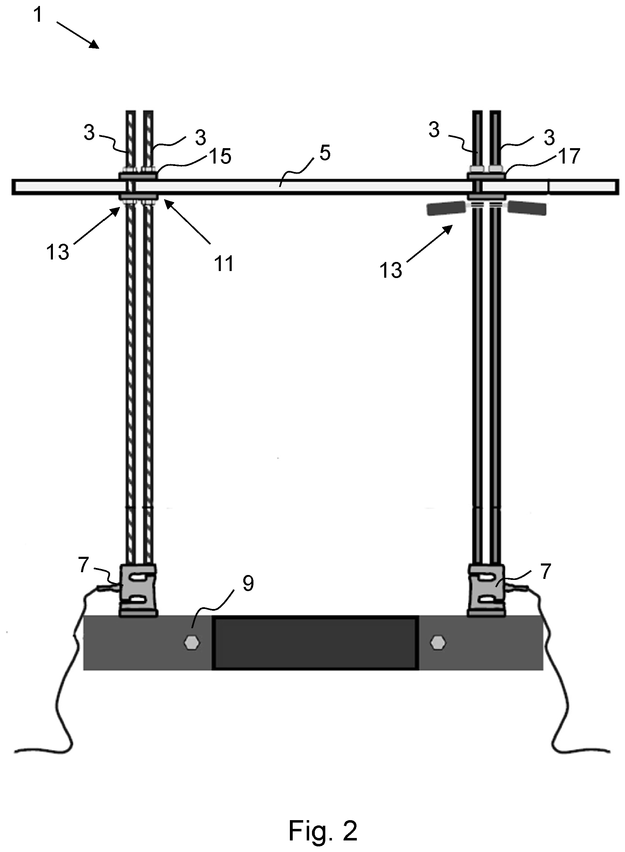

**Figure 2** shows an example of the muscle strength measuring device 1 having two rods 3 on each side, wherein the rods 3 on one of the sides are threaded and the rods 3 on the other side are smooth. On the side with the threaded rods 3, the locking arrangement 13 may comprise one or more screw-nuts and on the side with the smooth rods 3, the locking arrangement 13 may comprise one or more stop rings or spring clamps.

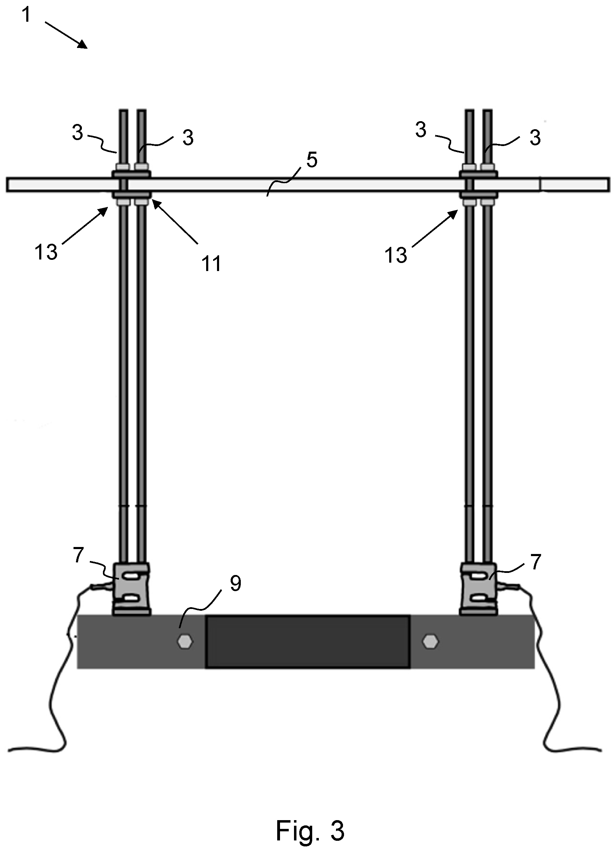

**Figure 3** shows an example of the muscle strength measuring device 1 having two rods 3 on each side, wherein all the rods 3 are smooth. The example in figure 3 thus shows the muscle strength measuring device 1 with four smooth rods 3. With this solution, the locking arrangement 13 may preferably comprise one or more stop rings or spring clamps.

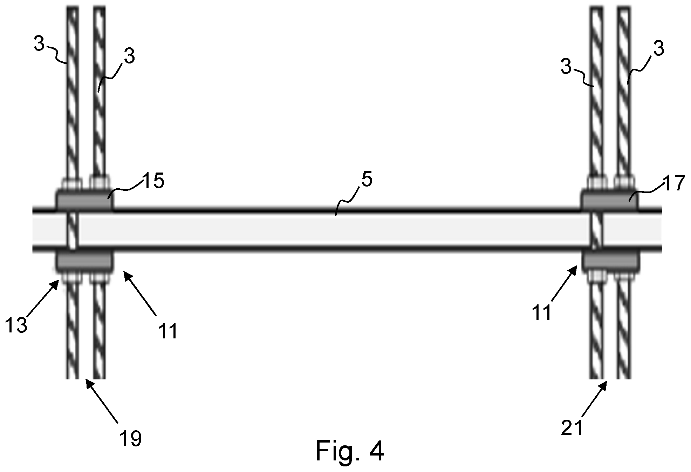

According to an embodiment, the muscle strength measuring device 1 comprises at least two rods 3 on each side as illustrated in **figure 4****.** The rods 3, which in this example are threaded, may comprise a first set of rods 19 and a second set of rods 21 arranged at a horizontal distance from each other. The rods in each of said first and second sets of rods 19, 21 may be arranged spaced apart so that a space is formed between them for receiving the barbell 5. The holding arrangement 11 may comprise a first holding device 15 arranged movable along the first set of rods 19, and a second holding device 17 arranged movable along the second set of rods 21, wherein each of the first and second holding devices 15, 17 defines an opening between the rods of said first and second sets of rods 19, 21 for receiving one end of the barbell 5.

The locking arrangement 13 may be arranged to lock the first and second holding devices 15, 17 against the first and second sets of rods 19, 21 respectively in any desired vertical position. At least one of the first set of rods 19 and the second set of rods 21 may be threaded and wherein the locking arrangement 13 is provided with at least one opening with corresponding threads. Having two rods 3, e.g. threaded rods, on each side where the barbell 5 is clamped is advantageous as it makes it very easy to remove the barbell 5.

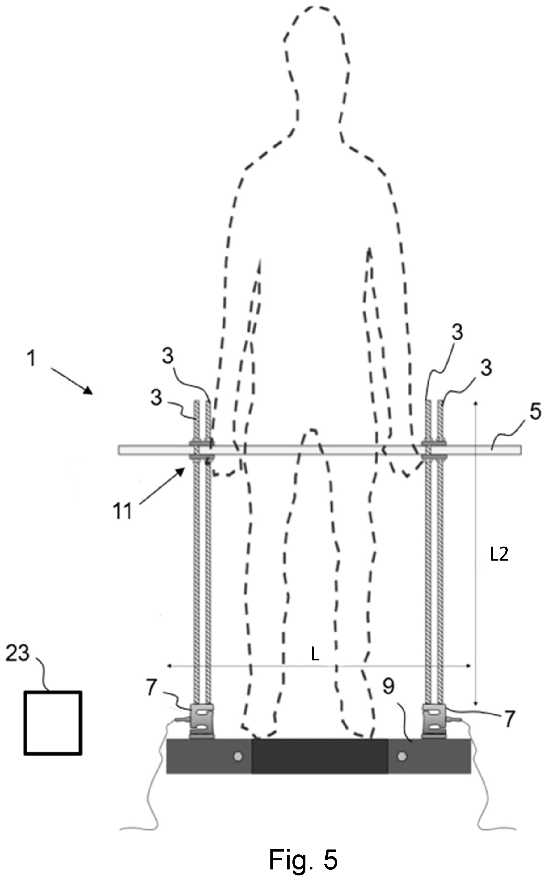

According to an embodiment the muscle strength measuring device 1 comprises one rod 3, i.e. a single rod, on each side and the barbell 5 may comprise one or more openings for receiving the rods 3. The single rod 3 may thus be arranged to centrally receive the barbell 5. **Figure 5** shows an example of the muscle strength measuring device 1 in a frontal view according to an embodiment. The figure 5 shows a test person standing on the base unit 9 ready to perform a test on the muscle strength measuring device 1. The muscle strength measuring device 1 may comprise the control unit 23 connected to the sensor units 7. The connection between the control unit 23 and the sensor units 7 may be wireless or wired. The control unit 23 may be configured to receive measured force data from the sensor units 7. The control unit 23 may further be configured to determine a zero-force crossing point, which defines the start of a test, based on the force transitioning from negative to positive. The control unit 23 may further be configured to determine the force at predefined time points based on the measuring data and the determined zero-force crossing point. The zero-force crossing point may be determined based on that a positive force is preceded by a negative force.

Each sensor unit 7 y comprises a load cell. The load cells are bi-directional load cells such that they measure both tension forces and compression forces.

With the embodiments described herein, force can be measured using a muscle strength measuring device 1 comprising two sensor units 7 connected in series with a plurality of rods 3, where a barbell 5 can be fixed at a desired lifting height for the test subject with millimetre precision.

The rods 3 may extend vertically from a base unit 9 and have a length L2, which in the example shown in Figure 5 is 1 meter. However, any other suitable length L2 of the rods 3 may be used. The sensor units 7 may be attached at their lower end to the base unit 9. The base unit 9 may have a length L of 1 meter, as shown in Figure 5, although other suitable lengths may also be employed.

In one embodiment, the base unit 9 comprises a wooden bar centrally attached to two shorter bars, each 50 cm in length. However, the base unit 9 may alternatively be made of metal or other structurally suitable materials, depending on the desired weight, durability, and application.

The use of load cells is advantageous, as they are significantly more cost-effective than force plates - typically around one fifth of the price. Moreover, the combination of load cells and rods 3, such as threaded rods, provides a lightweight and compact alternative to conventional setups involving force plates and poles. The rods 3 also occupy less physical space than traditional posts, thereby enhancing the portability and convenience of the muscle strength measuring device 1.

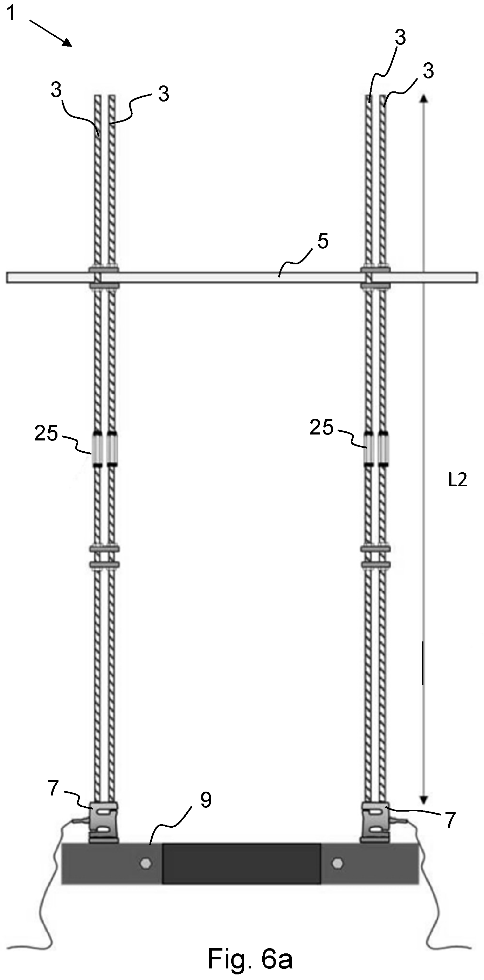

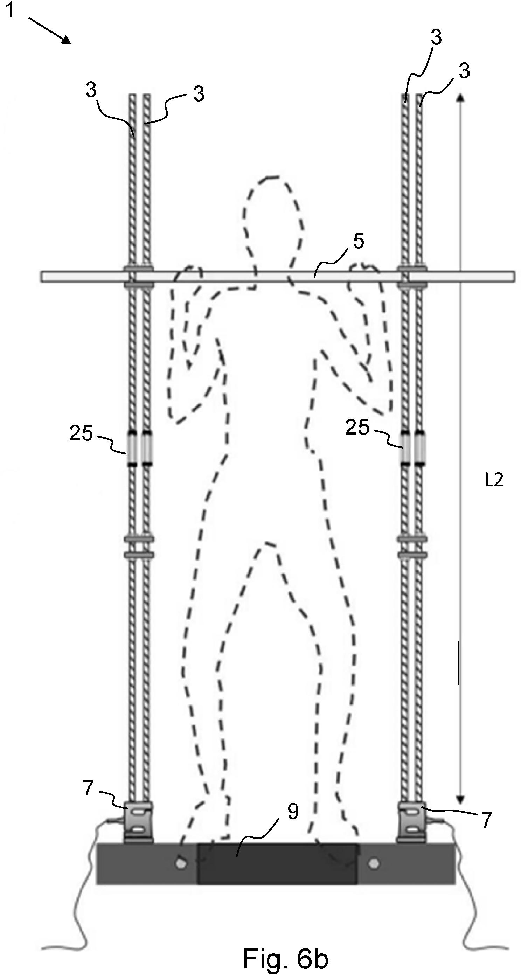

**Figure 6a** **and** **6b** shows examples of the muscle strength measuring device 1 wherein the rods 3 have been extended using one or more splicing nuts 25, e.g. union nuts. Figure 6b shows a test person standing on the base unit ready to perform a test. The length L2 of the rods may be 2 meters. However, any other suitable lengths L2 of the rods are possible.

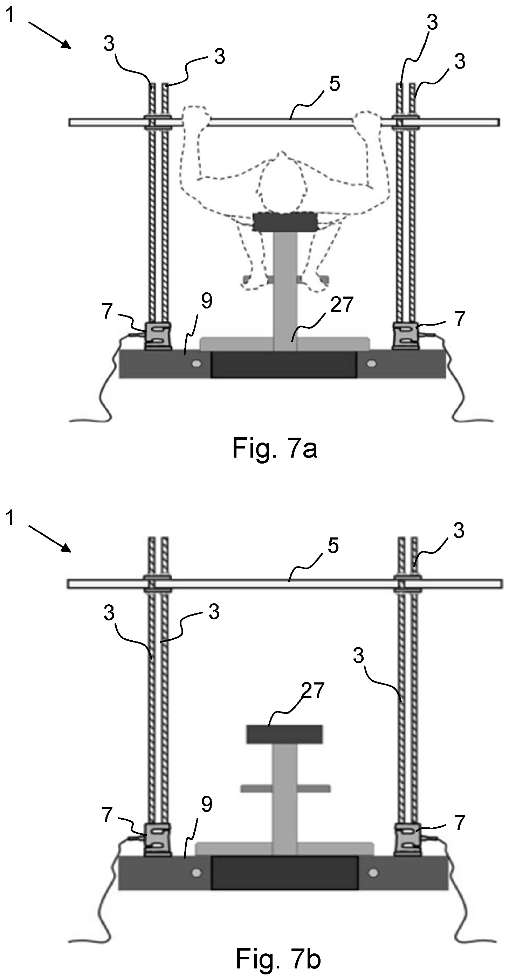

**Figure 7a and 7b** shows examples of an embodiment of the muscle strength measuring device 1. The muscle strength measuring device 1 may comprise a bench 27 which may be located on the base unit 9. The bench 27 may be a bench press training bench. In figure 7a the test person lies on the bench 27 ready to perform a test. The test person may start the test by laying on the bench 27 and pressing the barbell 5 upwards, i.e. pushing the barbell.

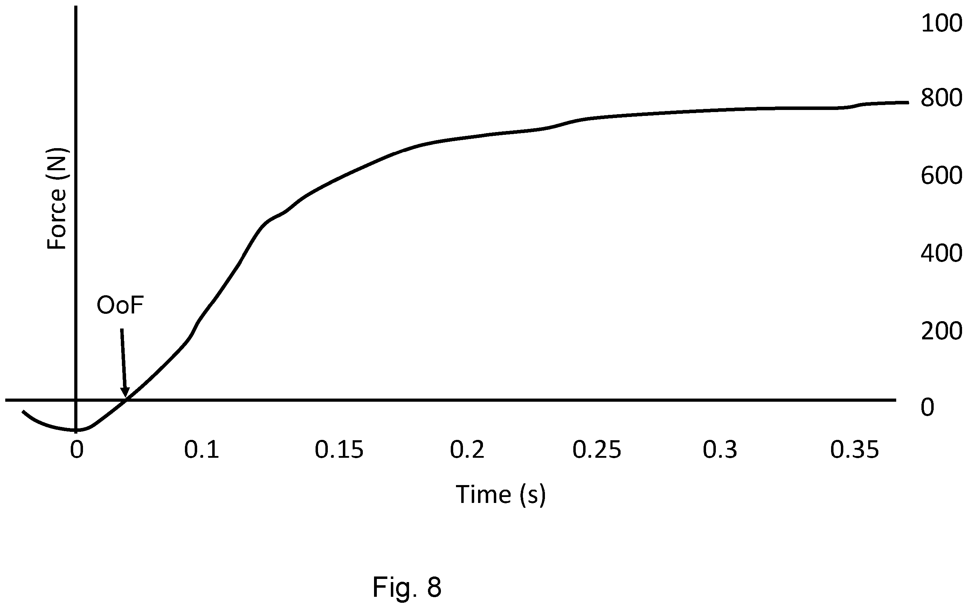

With the muscle strength measuring device 1 it is possible to accurately detect the zero-crossing point, i.e. zero-force crossing point. The zero-force crossing point, or on-set of force OoF, when used herein, can be defined as the point where the force passes zero, as illustrated in **figure 8****.** In an example scenario a test person stands on the base unit, adjusts and locks the barbell 5 against the rods 3 in any desired vertical position using the locking arrangement 13, and starts to pull or push. Force values may then be measured by the sensor units 7 and the control unit 23 may be adapted to determine the zero-force crossing point, which defines the start of a test, based on the force transitioning from negative to positive. The control unit 23 may then determine the force at predefined time points based on the measuring data and the determined zero-force crossing point. The force values may then automatically be reported by the control unit 23 at the predefined time points, e.g. at 50ms, 100ms, 150ms, 200ms and 250ms, to the test person. The muscle strength measuring device 1 enables measurement of maximal strength and explosive strength, i.e. the first 250ms of the test. It is therefore of great value to know when the test is initiated, which is why the zero-force crossing point is determined.

In an example scenario for a pull test, such as IMTP, a test person stands on the base unit, adjusts and locks the barbell 5 against the rods 3 in any desired vertical position using the locking arrangement 13, applies a light pushing force (opposite direction of the force to be tested) and then starts to pull at the start of test. Force values may then be measured by the sensor units 7 and the control unit 23 may be adapted to determine the zero-force crossing point, which defines the start of a test, based on the force transitioning from negative to positive."

As sensor units 7, e.g. bi-directional load cells, are used to measure both tension (push) and compression (pull), it allows to have the test person low force counter pre-tension muscle action (push if the test is pull and pull if the test is push), i.e., a compressive action when the test involves pulling, or a tensile action when the test involves pushing. This in turn allows for the precise detection of force onset - at zero force - when the force transitions from negative (pushing) to positive (pulling). The onset of force OoF can thus be determined with high precision, defined as the moment when the measured force transitions from negative to positive, corresponding to the zero-force crossing point. Furthermore, as two sensor units 7 are used it is possible to more reliably assess unilateral force development and thus asymmetries, i.e. differences in muscle strength between the right and left halves of the body. As force is measured with two sensor units 7, it is possible to distinguish between the left and right halves of the body.

Further advantages of the muscle strength measuring device 1 according to the invention include that it is cost-effective, lightweight, compact, and portable.

Additionally, the device enables the use of negative pre-tension to achieve a distinct zero-force crossing point, thereby allowing for more accurate detection of force onset. This is particularly important for determining force output at predefined time intervals during a test. Another advantage of the muscle strength measuring device 1 is that it allows for precise adjustment of the distance between the barbell 5 and the base unit 9, which enhances measurement reliability and facilitates testing in the subject's most biomechanically optimal lifting position.

The terminology used herein is for the purpose of describing particular aspects of the disclosure only and is not intended to limit the invention. As used herein, the singular forms "a", "an" and "the" are intended to include the plural forms as well, unless the context clearly indicates otherwise. Unless otherwise defined, all terms used herein have the same meaning as commonly understood by one of ordinary skill in the art to which this disclosure belongs.

It will be appreciated that the foregoing description and the accompanying drawings represent non-limiting examples of the device taught herein. As such, the device and techniques taught herein are not limited by the foregoing description and accompanying drawings. Thus, the present invention is not limited to the embodiments disclosed but may be varied and modified within the scope of the following claims. For example, the shapes of the rods, the holding arrangement, the locking arrangement, the barbell, the base unit, and the first- and second holding device may vary.

### Reference list

1. Muscle strength measuring device
3. Rods
5. Barbell
7. Sensor units
9. Base unit
11. Holding arrangement
13. Locking arrangement
15. First holding device
17. Second holding device
19. First set of rods
21. Second set of rods
23. Control unit
25. Splicing nuts
27. Bench
L. Length of base unit
L2. Length of rods

## Claims

1. A muscle strength measuring device (1) comprising:
- a barbell (5),
- a base unit (9),
- a plurality of rods (3) extending vertically from the base unit (9) and arranged to receive the barbell (5),
- two sensor units (7) arranged between the base unit (9) and the rods (3) and being configured to measure a force applied to the barbell (5), and
- a holding arrangement (11) for holding the barbell (5) in a fixed vertical position with respect to the base unit (9) during measuring of the force applied to the barbell (5), **characterized in that** the holding arrangement (11) comprises:
- a locking arrangement (13) movable along the rods (3) and arranged to lock the barbell (5) against the rods (3) in any desired vertical position to enable adjustment of the barbell (5) along the rods (3),
wherein each sensor unit (7) comprises a load cell, which load cells are bi-directional load cells such that they measure both tension forces and compression forces,
further comprising a control unit (23) connected to the sensor units (7), wherein the control unit (23) is configured to:
receive measured force data from the sensor units (7),
determine a zero-force crossing point, which defines the start of a test, based on the force transitioning from negative to positive, and
wherein the zero-force crossing point is determined based on that a positive force is preceded by a negative force.

2. The muscle strength measuring device (1) according to claim 1, the control unit (23) is configured to determine the force at predefined time points based on the measuring data and the determined zero-force crossing point.

3. The muscle strength measuring device (1) according to claim 1 or 2, wherein at least one of the rods (3) is threaded and wherein the locking arrangement (13) is provided with at least one opening with corresponding threads.

4. The muscle strength measuring device (1) according to claim 1 to 3, wherein said plurality of rods (3) comprises a first set of rods (19) and a second set of rods (21) arranged at a horizontal distance from each other, and the rods in each of said first and second sets of rods are arranged spaced apart so that a space is formed between them for receiving the barbell (5), and wherein the holding arrangement comprises:
- a first holding device (15) arranged movable along the first set of rods (19), and
- a second holding device (17) arranged movable along the second set of rods (21), wherein each of the first and second holding devices (15), (17) defines an opening between the rods of said first and second sets of rods (19), (21) for receiving one end of the barbell (5).

5. The muscle strength measuring device (1) according to claim 6, wherein the locking arrangement (13) is arranged to lock the first and second holding devices (15), (17) against the first and second sets of rods (19), (21) respectively in any desired vertical position.

6. The muscle strength measuring device (1) according to claim 4 or 5, wherein at least one of the first set of rods (19) and the second set of rods (21) are threaded and wherein the locking arrangement (13) is provided with at least one opening with corresponding threads.

7. The muscle strength measuring device (1) according to claim 1 or 2, wherein the barbell (5) comprises one or more openings for receiving the rods (3).
